# EUROPEAN PATENT SPECIFICATION

(11) **EP 1 420 704 B1**
(45) Date of publication and mention of the grant of the patent: **16.11.2005**
(21) Application number: 02716228.8
(22) Date of filing: 30.01.2002
(51) Int. Cl.: A61B 17/326

(54) **CIRCUMCISION CLAMP**
BESCHNEIDUNGSKLAMMER
PINCE A CIRCONCIRE

(30) Priority: 30.01.2001 MY 0100375
(43) Date of publication of application: 26.05.2004
(73) Proprietor: Circumvent BV, Hengelo (NL)
(72) Inventor: TEN HAVE, Henri Ferdinand, 12 JalanYapKwanSeng 50450 Kuala Lumpur (MY)
(74) Representative: Bates, Philip Ian
(86) International application number: PCT/IB2002/000272
(87) International publication number: WO 2002/060329

(56) References cited:
- DE-C- 546 440
- US-A- 2 296 594
- US-A- 2 484 132
- US-A- 3 072 126
- US-A- 5 649 933

## Description

This invention relates to clamps for use in circumcision operations and particularly to clamps for clamping the prepuce of the patient in a desired position for excision.

### BACKGROUND TO THE INVENTION

EP-B-0637225 describes a lightweight circumcision clamp of unitary construction comprising a clamping ring of annular shape associated with a tubular support having an open distal end and a flared proximal end section for reception of the glans penis and external envelopment by the prepuce, the clamping ring and the tubular support being movable relative to each other between an open position defining a prepuce- receiving gap and a closed position in which the gap is closed, and locking means for locking the clamping means in the closed position, wherein the clamping ring is displaceably mounted on and carried by the tubular support and the distal end of the tubular support permits voiding of urine and enlargement of the penis, such that the clamp may be worn for a few days.

In a preferred form of the clamp the locking means comprises a pair of arms positioned at diametrically opposed sides of the locking ring at one end and having a locking formation at the other end for engagement with a complimentary locking formation on the tubular support. At about their mid-point a stabilizing and leveraging strut hingedly connects each arm to the tubular support.

In practice, the clamp is formed in two parts, the tubular support forming one part and the second part comprising the locking ring, the pair of arms, the struts and a second tubular member to which the struts are connected and which carries the complimentary locking formation. The second tubular member fits over the distal end of the tubular support and is locked thereto in such a way that the two parts cannot be separated without breaking the clamp.

This clamp is a great improvement over previously proposed clamps but has drawbacks in that it is not easy to use. The arrangement of the arms and struts cause the clamping ring to be permanently biased towards the flared end of the tubular support so that, in order that the flared end can be fitted over the glans penis and the prepuce pulled over the outside of the flared end, which cannot easily be done with one hand, the prepuce receiving gap has to be maintained as large as possible by a second person who retracts the clamping ring. With children who require only a small clamp there is virtually no room for a second person's hands and the person fitting the clamp usually ends up pulling the prepuce through a small gap using tweezers. This can, of course, be very distressful for the patient, even under anaesthetic. It is also very difficult to position the prepuce so that the incision can be made in exactly the right place. The locking formation, moreover requires considerable force to engage the two cooperating parts and this can cause the hands to tremble thus causing increased trauma to the patient. Removal of the clamp is also difficult and can cause pain to the patient.

An improved circumcision clamp would therefore be a great advantage to persons carrying out circumcision operations.

### SUMMARY OF THE INVENTION

According to the invention, a circumcision clamp is formed in two parts, the first part comprising a tubular support member, open at both ends, and having a flared proximal end for receiving the glans penis of the patient and over which the prepuce of the patient can be stretched to a desired degree, and the second part comprising a lockable clamping ring that can be slid over the tubular member and the prepuce stretched thereover, at least as far as the desired position of excision, and locked to clamp the prepuce to the outer surface of the tubular member sufficiently tightly to stem bleeding

In the simplest form of the clamp, the clamping ring has a pair of diametrically arranged hinged arms with one part of a locking arrangement at their free ends and a second part of the locking arrangement is formed on the outer surface of the tubular member.

However, in a preferred form of the clamp, the distal end of the tubular member carries a flange and the second part of the clamp includes a second ring having an opening that will fit over the flange such that the second part can be twisted relative to the tubular member to lock the second part to the tubular member. Such locking may be effected by means of screw threads on the flange and the second ring or a bayonet type arrangement.

Preferably, however, the flange is not circular and the second ring has an opening of corresponding shape, such that, after the flange has been passed through the opening, relative rotation of the two parts causes the ring to engage underneath the flange. The clamping ring and the second ring are connected by a pair of diametrically opposed arms hingedly attached at each end to a ring and each having a hinge and a locking arrangement along their length.

Preferably, in order to ensure that there is sufficient space between the arms and the prepuce at the position of excision to insert a scalpel, the arms are provided with a projection on their inner surface that, when they are locked may contact the surface of the tubular member to limit their movement towards the tubular member.

The hinges in the arms, preferably those connecting the arms to the clamping ring, are preferably formed so they can be easily broken or cut, for example, by scissors or shears, when it is desired to remove the clamp.

The clamp of the invention is made from a plastics material, preferably, a poly acetal resin, such as "DELRIN" (Registered Trade Mark), preferably by injection moulding. The material is preferably transparent so that the penis of the patient can be inspected through the tubular member to check for signs of infection, retention of urine or other anomalies.

In use of the clamp, the prepuce of the patient is rolled back and the flared end of the tubular portion is positioned over the glans penis, the prepuce is then rolled back over the outer surface of the tubular member and marked at the proposed position of the cut. The clamping ring is then placed over the tubular member, and, in the preferred form of the clamp twisted through, say 90 degrees, to lock the second part to the tubular member. At this time the clamping ring has no contact with the prepuce. The clamping ring is then positioned over the prepuce and the locks closed to clamp the prepuce against the surface of the tubular member. This can be done by a single person, without difficulty. The operation can then be carried out The clamp is left in position after the operation until the wound has healed, after which it is removed by cutting through the hinges between the arms and the clamping ring and lifting the clamp away from the penis.

### DESCRIPTION OF PREFERRED EMBODIMENT

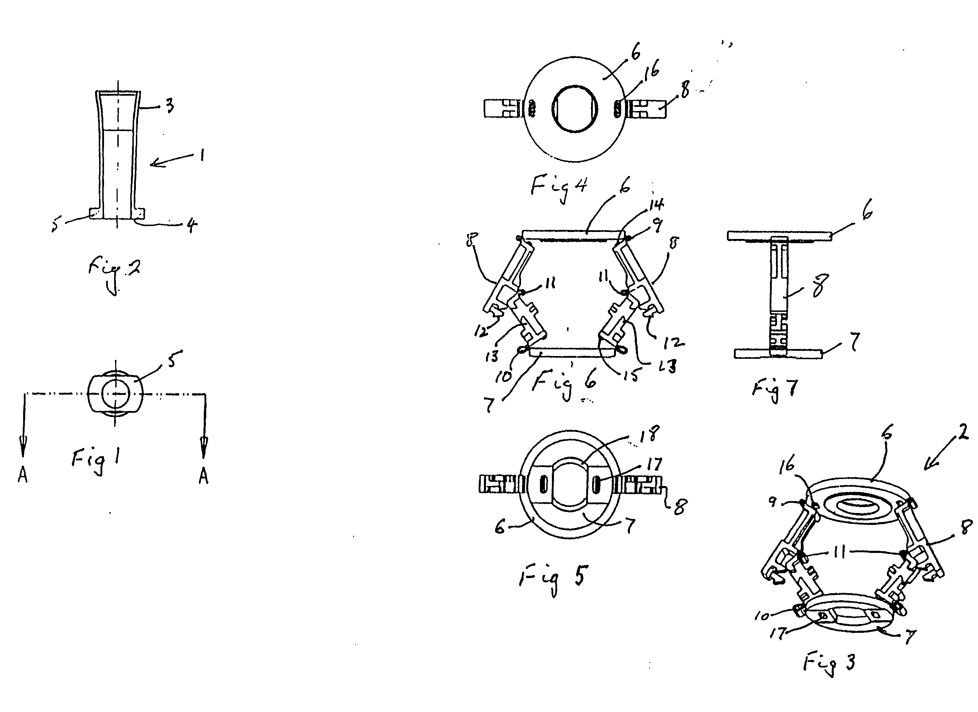

The invention will now be described in greater detail, by way of example, with reference to the drawings, in which
Fig. 1 is a distal end view of the tubular support member of a preferred form of clamp according to the invention;
Fig.2 is a section on line A-A in Fig. 1;
Fig. 3 is a perspective view of the clamping portion of the clamp of Fig. 1;
Fig. 4 is a distal end view of the clamping portion shown in Fig. 3;
Fig. 5 is a proximal end view of the clamping portion shown in Fig. 3;
Fig. 6 is a side view of the clamping portion shown in Fig. 3; and
Fig. 7 is a second side view turned through 90 degrees of the clamping portion shown in Fig. 3

As shown in the drawings the preferred form of clamp is made from plastics material and comprises two parts, a tubular support member 1 and a clamping portion 2.

The tubular member 1 is of circular cross-section and open at both ends. At its proximal end 3, member 1 is flared to receive the glans penis of a patient. The prepuce of the patient is pulled over the flared end 3. At its distal end 4, member 1 has an external flange 5 which has a generally rectangular configuration but with rounded ends.

The clamping portion 2 comprises a clamping ring 6 connected to a second ring 7 by arms 8.

Arms 8 are integral with rings 6 and 7 and are hinged thereto at 9 and 10, respectively. Arms 8 also have a lockable hinge 11 at a position about mid-way along their length. The locking arrangement of hinges 11 comprises a detent member 12 that engages under a lip 13. On their inner surfaces arms 8 have a projection 14, which, when the hinges 11 are locked contacts the surface of the tubular member 1 to limit inward movement of arms 8 and ensure that clearance beneath the arms 8 is maintained to allow adequate space for insertion of a scalpel. On their end surfaces arms 8 carry projections 14, 15 for engagement in apertures 16,17 in the clamping ring 6 and second ring 7, respectively, when hinges 11 are locked, to prohibit relative rotational movement of rings 6 and 7.

Second ring 7 has a central opening 18 of the same shape as the external configuration of flange 5. When ring 7 is passed over flange 5 and turned through 90 degrees, clamping portion 2 becomes locked to tubular member 1. In this situation locking of hinges 11 forces clamping ring 6 over the prepuce stretched over the flanged end 3 of tubular member 1 to clamp it in position for the operation.

The lockable hinges 11 in the arms 8 can be very simply locked without applying undue strain to the penis of the patient using forceps or tweezers.

The clamp of the invention will be made in various sizes to cater for all ages of patient from little babies to adults with different sizes of penis.

## Claims

1. A two-part circumcision clamp, the first part comprising a tubular support member (1), open at both ends, and having a flared proximal end (3) for receiving the glans penis of the patient and over which the prepuce of the patient can be stretched to a desired degree, and the second part separated from the first part, comprising a lockable clamping ring (3) that can be slid over the tubular member (1) and the prepuce stretched thereover, at least as far as the desired position of excision, and locked to clamp the prepuce to the outer surface of the tubular member (1) sufficiently tightly to stem bleeding.

2. A clamp according to claim 1, wherein the distal end (4) of the tubular member (1) carries a flange (5) and the second part of the clamp includes a second ring (7) having an opening (19) that will fit over the flange (5) such that the second part can be twisted relative to the tubular member (1) to lock the second part to the tubular member (1), the clamping ring (6) and the second ring (7) being connected together by hinged arms (8).

3. A clamp according to claim 2, wherein the flange (5) is not circular and the opening (19) in the second ring (7) is of corresponding shape, such that, after the flange (5) has been passed through opening (19), relative rotation of the two parts causes the ring (7) to engage underneath the flange (5), the clamping ring (6), the second ring (7) being connected by a pair of diametrically opposed arms (8) hingedly attached at each end to said rings (6,7) and each having a hinge (11) and a locking arrangement (12,13) along their length.

4. A clamp according to claim 2 or claim 3, wherein the arms (8) are provided with a projection (14) on their inner surface, which, when the arms (8) are locked may contact the surface of the tubular member (1) to limit their movement towards the tubular member (1).

5. A clamp according to any one of claims 2 to 4, wherein the hinges (9) connecting the arms to the clamping ring (6), are formed so they can be easily broken or cut, when it is desired to remove the clamp.

6. A clamp according to any one of claims 3 to 5, wherein the ends of arms (8) carry projections (14,15) for engagement in apertures (16,17) in clamping ring (6) and second ring (7) when hinges (11) are locked to prevent relative rotational movement of rings (6,7).

7. A clamp according to any one of claims 1 to 6 made from a plastics material.

8. A damp according to claim 7 made from polyacetal resin.

9. A clamp according to claim 7 or claim 8 made from a transparent plastics material.

## Patentansprüche

1. Zweiteilige Beschneidungsklammer, wobei der erste Teil ein röhrenförmiges Stützelement (1) umfasst, das an beiden Enden offen ist und ein aufgeweitetes proximales Ende (3) zur Aufnahme der Glans penis des Patienten aufweist und über das das Praeputium des Patienten bis zu einem gewünschten Grad gestreckt werden kann, und wobei der zweite Teil, der von dem ersten Teil getrennt ist, einen verriegelbaren Klemmring (3) umfasst, der über das röhrenförmige Element (1) und das darüber gestreckte Praeputium wenigstens bis zu der gewünschten Schnittposition geschoben und verriegelt werden kann, um das Praeputium fest genug zum Stillen der Blutung auf die Außenfläche des tubulären Elementes (1) zu klemmen.

2. Klammer nach Anspruch 1, wobei das distale Ende (4) des tubulären Elementes (1) einen Flansch (5) trägt und der zweite Teil der Klammer einen zweiten Ring (7) mit einer Öffnung (19) beinhaltet, die über den Flansch (5) passt, so dass der zweite Teil relativ zum tubulären Element (1) gedreht werden kann, um den zweiten Teil auf dem tubulären Element (1) zu verriegeln, wobei der Klemmring (6) und der zweite Ring (7) durch angelenkte Arme (8) miteinander verbunden sind.

3. Klammer nach Anspruch 2, wobei der Flansch (5) nicht kreisförmig ist und die Öffnung (19) im zweiten Ring (7) eine entsprechende Gestalt hat, so dass die relative Rotation der beiden Teile nach der Passage des Flansches (5) durch die Öffnung (19) bewirkt, dass der Ring (7) unter dem Flansch (5) eingreift, wobei der Klemmring (6) und der zweite Ring (7) durch ein Paar diametral gegenüber liegender Arme (8) verbunden sind, die an jedem Ende an den genannten Ringen (6, 7) angelenkt sind und jeweils ein Gelenk (11) und eine Verriegelungsanordnung (12, 13) entlang ihrer Länge haben.

4. Klammer nach Anspruch 2 oder Anspruch 3, wobei die Arme (8) mit einem Vorsprung (14) an ihrer Innenfläche versehen sind, der, wenn die Arme (8) verriegelt sind, mit der Oberfläche des tubulären Elementes (1) in Kontakt kommen kann, um deren Bewegung in Richtung auf das tubuläre Element (1) zu begrenzen.

5. Klammer nach einem der Ansprüche 2 bis 4, wobei die die Arme mit dem Klemmring (6) verbindenden Gelenke (9) so geformt sind, dass sie leicht zerbrochen oder zerschnitten werden können, wenn die Klammer entfernt werden soll.

6. Klammer nach einem der Ansprüche 3 bis 5, wobei die Enden von Armen (8) Vorsprünge (14, 15) für den Eingriff in Löcher (16, 17) in dem Klemmring (6) und dem zweiten Ring (7) tragen, wenn die Gelenke (11) verriegelt sind, um eine relative Drehbewegung der Ringe (6, 7) zu verhindern.

7. Klammer nach einem der Ansprüche 1 bis 6, die aus einem Plastikmaterial gefertigt ist.

8. Klammer nach Anspruch 7, die aus Polyacetalharz gefertigt ist.

9. Klammer nach Anspruch 7 oder 8, die aus einem transparenten Plastikmaterial gefertigt ist.

## Revendications

1. Clamp de circoncision en deux parties, la première partie comprenant un membre de support tubulaire (1), ouvert aux deux extrémités, et ayant une extrémité proximale évasée (3) pour recevoir le gland du pénis du patient et sur laquelle le prépuce du patient peut être étiré à un degré désiré, et la deuxième partie, séparée de la première partie, comprenant un anneau de serrage verrouillable (3) que l'on peut faire glisser sur le membre tubulaire (1) et le prépuce étiré dessus, au moins jusqu'à la position d'excision désirée, et verrouillé pour serrer au moins suffisamment fort le prépuce à la surface externe du membre tubulaire (1) pour arrêter un saignement.

2. Clamp selon la revendication 1, où l'extrémité distale (4) du membre tubulaire (1) porte une bride (5) et la deuxième partie du clamp comprend un deuxième anneau (7) ayant une ouverture (19) qui s'adaptera au-dessus de la bride (5) de telle sorte que l'on peut tourner la deuxième partie par rapport au membre tubulaire (1) pour verrouiller la deuxième partie sur le membre tubulaire (1), l'anneau de serrage (6) et le deuxième anneau (7) étant raccordés ensemble par des bras à charnière (8).

3. Clamp selon la revendication 2, où la bride (5) n'est pas circulaire et l'ouverture (19) dans le deuxième anneau (7) est de forme correspondante, de telle sorte qu'après que l'on a fait passer la bride (5) à travers l'ouverture (19), une rotation relative des deux parties fait que l'anneau (7) s'engage sous la bride (5), l'anneau de serrage (6), le deuxième anneau (7) étant raccordés par une paire de bras diamétralement opposés (8) attachés par des charnières à chaque extrémité desdits anneaux (6, 7) et ayant chacun une charnière (11) et un arrangement de verrouillage (12, 13) le long de leur longueur.

4. Clamp selon la revendication 2 ou la revendication 3, où les bras (8) sont pourvus d'une saillie (14) sur leur surface interne, qui, lorsque les bras (8) sont verrouillés, peut entrer en contact avec la surface du membre tubulaire (1) afin de limiter leur mouvement vers le membre tubulaire (1).

5. Clamp selon l'une quelconque des revendications 2 à 4, où les charnières (9) raccordant les bras à l'anneau de serrage (6) sont formées de manière à être facilement brisées ou coupées lorsque l'on désire retirer le clamp.

6. Clamp selon l'une quelconque des revendications 3 à 5, où les extrémités des bras (8) portent des saillies (14, 15) destinées à s'engager dans des orifices (16, 17) dans l'anneau de serrage (6) et le deuxième anneau (7), lorsque les charnières (11) sont verrouillées, pour empêcher le mouvement rotatif relatif des anneaux (6, 7).

7. Clamp selon l'une quelconque des revendications 1 à 6, fabriqué en matière plastique.

8. Clamp selon la revendication 7, fabriqué en résine polyacétal.

9. Clamp selon la revendication 7 ou la revendication 8, fabriqué en matière plastique transparente.
